(19) Europäisches Patentamt
European Patent Office
Office européen des brevets

(11) Numéro de publication: **0 345 163**
**A1**

(12) **DEMANDE DE BREVET EUROPEEN**

(21) Numéro de dépôt: **89401510.6**

(22) Date de dépôt: **01.06.89**

(51) Int. Cl.[4]: **C 07 C 103/78**
A 61 K 49/04

(30) Priorité: **02.06.88 FR 8807369**

(43) Date de publication de la demande:
**06.12.89 Bulletin 89/49**

(84) Etats contractants désignés:
**AT BE CH DE ES FR GB GR IT LI LU NL SE**

(71) Demandeur: **GUERBET S.A.**
**15, rue des Vanesses Z.A.C. Paris Nord II**
**F-93420 Villepinte (FR)**

(72) Inventeur: **Schaefer, Michel**
**4, rue Francois-Girardon**
**F-91380 Chilly-Mazarin (FR)**

**Dugast-Zrihen, Maryse**
**107, rue Bobillot**
**F-75013 Paris (FR)**

**Guillemot, Michel**
**3, rue Lalo**
**F-75116 Paris (FR)**

**Doucet, Didier**
**13, rue Amédée Dunois**
**F-93190 Livry-Gargan (FR)**

**Meyer, Dominique**
**23, rue du Tage**
**F-75013 Paris (FR)**

(74) Mandataire: **Le Guen, Gérard et al**
**CABINET LAVOIX 2, place d'Estienne d'Orves**
**F-75441 Paris Cédex 09 (FR)**

Revendications pour l'Etat contractant suivant: ES.

(54) **Nouveau composé triiodobenzénique non ionique iodé, son procédé de préparation et produit de contraste le contenant.**

(57) L'invention a pour objet un composé de formule

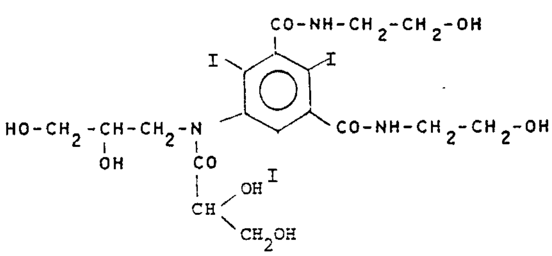

I

Ce composé est utilisable comme produit de contraste.

e.

EP 0 345 163 A1

## Description

## Nouveau composé triiodobenzénique non ionique iodé, son procédé de préparation et produit de contraste le contenant

La présente invention concerne des composés utilisables dans des produits de contraste pour la radiographie.

On utilise depuis longtemps comme produits de contraste des composés iodobenzéniques présentant sur le noyau benzénique plusieurs atomes d'iode, en général 3 atomes d'iode par noyau bénzénique, et divers autres substituants. Ces autres substituants sont des groupes pharmacologiquement acceptables qui permettent l'administration des composés chez l'homme et les animaux. Ces substituants sont d'une manière générale choisis, d'une part, pour conférer aux composés une hydrosolubilité suffisante en vue de l'administration de ces composés en solution aqueuse et, d'autre part, pour conférer à ces composés une tolérance suffisante par l'organisme humain.

A cet effet, on a proposé des structures non-ioniques, c'est-à-dire des dérivés iodobenzéniques possédant des substituants non-ioniques.

Ainsi, dans le brevet FR-A-2 053 037, on a proposé des composés carbamoyl iodobenzéniques contenant au total au moins un groupe N-hydroxy alcoyle et au moins deux groupes hydroxy.

Un composé illustrant cette classe est le métrizamide qui s'avère être toutefois d'une stabilité limitée.

La présente invention vise à fournir de nouveaux composés non-ioniques qui soient bien tolérés par l'organisme humain, très stables en solution aqueuse, qui présentent une forte solubilité dans l'eau et qui en solution aqueuse présentent une faible viscosité.

A cet effet, la présente invention a pour objet le composé de formule :

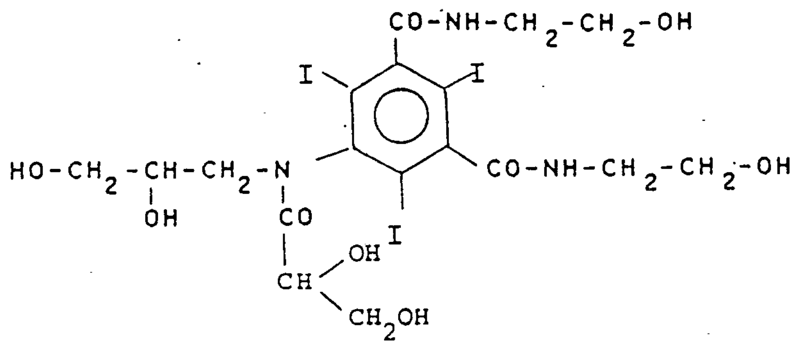

I

Le composé de formule I peut être préparé par un procédé consistant

a) à faire réagir une amine de formule

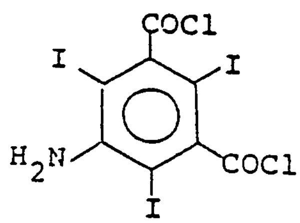

II

avec un chlorure d'acide de formule R'COCl, R' désignant un groupe $-\underset{OH}{CH}-CH_2OH$ dont les groupes hydroxy sont protégés, de façon à obtenir un composé de formule

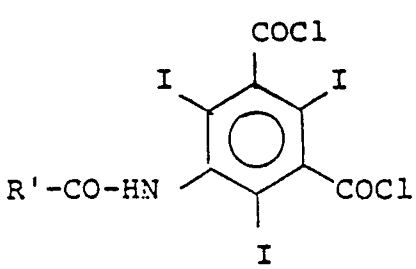

III

b) à faire réagir sur le composé de formule III l'éthanolamine de façon à obtenir un composé de formule

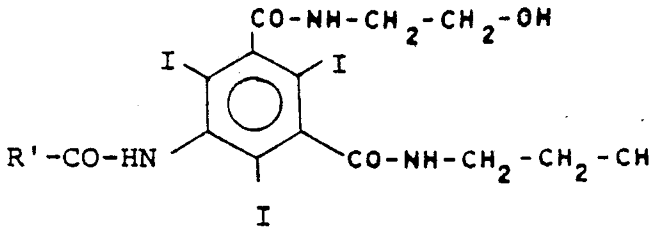

IV

puis soit

c) à faire réagir sur le composé de formule IV du 1-chloro-2,3-propanediol

et

d) à éliminer les groupes protecteurs du groupe R′

soit

c′) à déprotéger le composé de formule IV et

d′) à faire réagir sur le composé déprotégé le 1-chloro-2,3-propanediol.

La présente invention a également pour objet des produits de contraste qui comprennent au moins un composé de formule I.

Ces produits de contraste sont utilisés chez l'homme et les animaux à des fins radiologiques.

La forme pharmaceutique préférée des produits de contraste selon l'invention est constituée par des solutions aqueuses des composés.

Les solutions aqueuses contiennent généralement un total de 5 à 100 g de composés pour 100 ml et la quantité injectable de telles solutions peut varier généralement de 1 à 1000 ml.

Les solutions aqueuses des composés de formule I peuvent également contenir certains additifs comme :

- du chlorure de sodium à des concentrations comprises entre 0,1 et 10 mM/l
- de l'EDTA disodique à des concentrations comprises entre 0,1 et 2 mM/l
- du citrate de sodium à des concentrations comprises entre 0,1 et 10 mM/l
- de l'héparine à des doses comprises entre 10 et 100 unités pour 100 ml de solution.

Ces compositions peuvent être administrées par toutes les voies classiquement utilisées pour les produits de contraste non ioniques iodés. Ainsi elles peuvent être administrées par voie entérale ou parentérale (voir intraveineuse, intraartérielle, opacification des cavités) et en particulier dans l'espace sous-arachnoidien.

On donnera ci-après un exemple de composition selon la présente invention.

Composition

| | |
|---|---|
| Composé de l'exemple 1 | 65 g |
| Eau pour préparation injectable QSP | 100 ml |

EXEMPLE 1

Préparation du 5-[2,3-dihydroxy-N-(2,3-dihydroxypropyl) propionamido]-N′,N″-bis-(2-hydroxy-éthyl) -2,4,6-triiodoisophtalamide.

a) Préparation du dichlorure de 5-[2,2-diméthyl-1,3-dioxolanne-4-carboxamido]-2,4,6-triiodoisophtaloyle

54,3 g de chlorure de 5-amino-2,4,6-triiodo isophtaloyle (0,091 mole) sont dissous dans 200 ml de DMAC auxquels sont ajoutés 30 g (0,182 mole) du chlorure de l'acide 2,2-diméthyl-1,3 dioxolanne-4-carboxylique. Le milieu réactionnel est maintenu 24 heures sous argon à température ambiante. Le DMAC est évaporé sous vide. L'huile obtenue est versée sur de l'eau bi carbonatée et extraite avec 300 ml d'acétate d'éthyle. La phase organique est lavée avec 150 ml d'eau glacée, décantée, séchée et concentrée à sec. Le produit est recristallisé dans 100 ml de $CH_2Cl_2$. Après filtration, 45 g de solide sont obtenus.

| | | | |
|---|---|---|---|
| Rdt : | 68 % | | |
| CCM | silice (60F254) $CH_2Cl_2$ | Rf : | 0,19 |
| | silice (60F254) éther/éther de pétrole | Rf : | 0,52 |

| | | | | |
|---|---|---|---|---|
| % I : | 51,2 | (trouvé) - | 52,6 | (théorie) |
| % Cl : | 9,9 | (trouvé) - | 9,8 | (théorie) |
| RMN : | ($CDCl_3$ + DMSO) | | | |

Deux singulets (6H) à 1,5 et 1,7 ppm; multiplet mal résolu entre 4,1 et 4,8 ppm (3H) ; 1 pic échangeable avec $D_2O$ à 9,1 ppm (NH).

b) Préparation du 5-[2,2-diméthyl-1,3-dioxolanne-4-carboxamido]-N′, N″-bis-(2-hydroxyéthyl)-2,4,6-triiodo isophtalamide

105 g du produit obtenu en a (0,145 mole) sont dissous dans une solution de 400 ml de DMAC et 77ml (0,551 mole) de triéthylamine.

35 g d'éthanolamine (0,57 mole) sont additionnés goutte à goutte au milieu réactionnel.

Le réaction est agitée 3 heures à température ambiante. Le chlorhydrate de triéthylamine est séparé par filtration et le DMAC est évaporé sous vide. Le produit est purifié sur silice silanisée (élution eau puis eau/méthanol 8/1). 84 g de produit sont obtenus.

| | |
|---|---|
| Rdt : | 75% |
| % I : | 48.1 (trouvé) - 49,3 (théorie) |
| RMN | (DMSO) : |

Deux singulets à 1,4 et 1,5 ppm (6H) ; massif entre 3,1 et 3,8 ppm (8H) ; multiplet entre 4 et 4,7 ppm (3H) ; 1 pic élargi centré à 5,4 ppm (OH) échangeable avec $D_2O$.

| | | | |
|---|---|---|---|
| CCM (Silice 60F254) : | $CH_2Cl_2$/ méthanol 9/1 | Rf: | 0,28 |
| | $CH_2Cl_2$/ méthanol 8/2 | Rf: | 0,71 |

c) Préparation du 5-[2,3-dihydroxy-N-(2,3-dihydroxypropyl) propionamido]-N′,N″-bis-(2-hydroxyéthyl)-2,4,6-triiodoisophtalamide.

A une suspension de 100 g de composé obtenu en b (0,130 mole), dans 300 ml d'éthylène glycol sont ajoutés goutte à goutte 115 ml de méthylate 4N (0,46 mole) à 60°C puis 64,5 g de 1 chloro-2,3-propanediol (0,585 mole).

Après 6 heures à 60°C, 50 ml de méthylate 4N (0,2 mole) et 21,5 g de 1-chloro-2,3-propanediol (0,2 mole) sont ajoutés à nouveau. L'ensemble est maintenu une nuit à 60°C. Les sels minéraux sont éliminés par filtration. L'éthylène glycol est évaporé sous vide. Le résidu de distillation est repris avec 500 ml d'eau et 250 ml d'HCl 5N et laissé une nuit sous agitation à température ambiante. Le milieu réactionnel est concentré sous vide et solubilisé dans 500 ml d'éthanol. Les sels minéraux sont éliminés par filtration. L'éthanol est avaporé sous vide, le résidu est recristallisé dans 1 litre d'alcool isopropylique. Le précipité est filtré et purifié par HPLC (RP 18 (élution eau). Rdt total (alkylation-déprotection-purification) = 56,5%

| | | | | | |
|---|---|---|---|---|---|
| 1) | CCM | silice (60F254)MeOH/$CH_2Cl_2$/ $NH_4OH$ 4/6/0,1 | Rf: | 0,28 | |
| 2) | % I : | 46,7 | (trouvé) - | 47,2 | (théorie) |
| 3) | RMN : | (DMSO) | | | |

Massif, mal résolu, centré à 3,3 ppm (16 H); pic à 4,5 ppm (OH), échangeable à $D_2O$ (6 H); pic à 8,4 ppm (NH) échangeable à $D_2O$ (2 H).

(4) HPLC Hypersil C 8 5u 15 cm

| | | | | |
|---|---|---|---|---|
| Tampon pour | $NaH_2PO_4$ | 0,01 | M | 97 % |
| | MeOH | | | 3 % |
| Pureté | 96 % | | | |

**Revendications**

1. Composé de formule

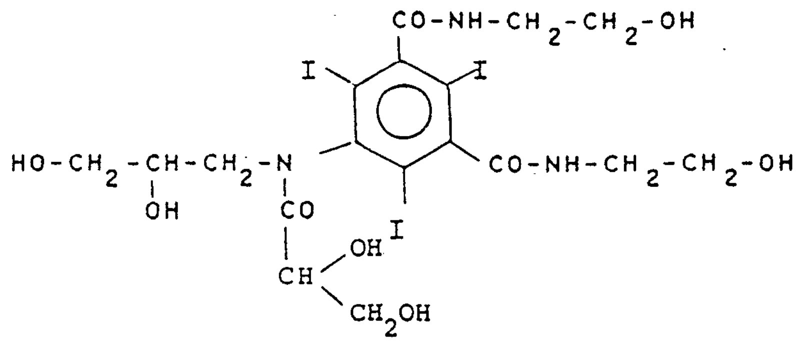

I

2. Produit de contraste caractérisé en ce qu'il contient au moins un composé selon la revendication 1.

3. Produit de contraste selon la revendication 2, caractérisé en ce qu'il est constitué par une solution aqueuse du ou des composés.

4. Procédé de préparation d'un composé selon la revendication 1 caractérisé en ce qu'il consiste

a) à faire réagir une amine de formule

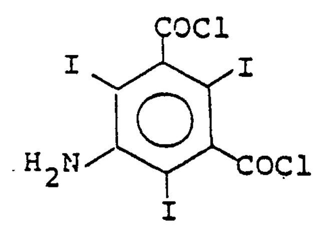

II

avec un chlorure d'acide de formule R'COCl, R'désignant un groupe $-CHOH-CH_2OH$ dont les groupes hydroxy sont protégés, de façon à obtenir un composé de formule

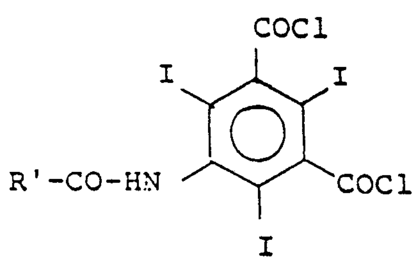

III

b) à faire réagit sur le composé de formule III l'éthanolamine de façon à obtenir un composé de formule

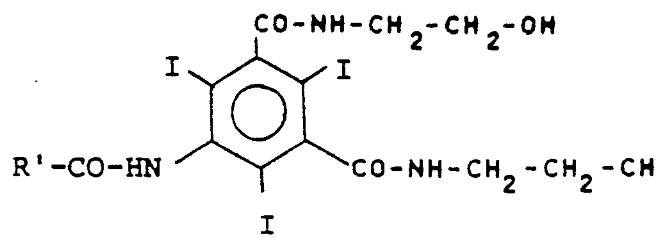

IV

puis soit

c) à faire réagir sur le composé de formule IV du 1-chloro-2,3-propanediolet

d) à éliminer les groupes protecteurs du groupe R′

soit

c′) à déprotéger le composé de formule IV et

d′) à faire réagir sur le composé déprotégé le 1-chloro-2,3-propanediol.

**Revendications pour l'Etat contractant suivant : ES**

1. Procédé de préparation d'un composé de formule

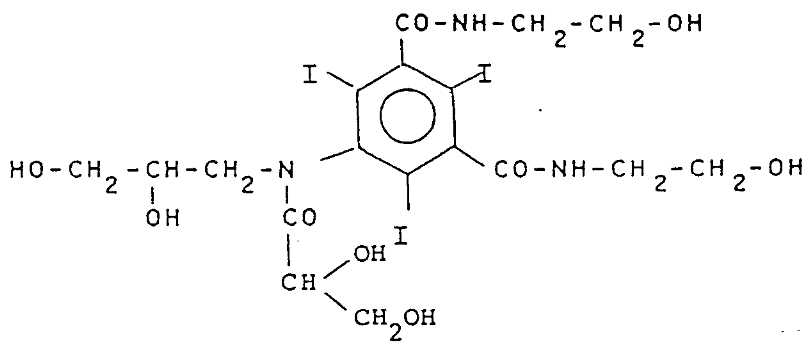

I

caractérisé en ce qu'il consiste

a) à faire réagir une amine de formule

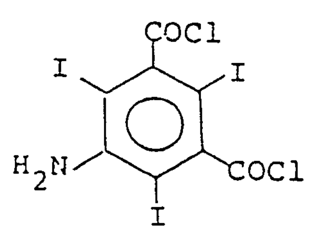

II

avec un chlorure d'acide de formule R′COCl, R′ désignant un groupe $-CHOH-CH_2OH$ dont les groupes hydroxy sont protégés, de façon à obtenir un composé de formule

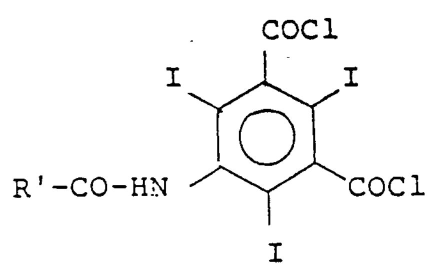

III

b) à faire réagir sur le composé de formule III l'éthanolamine de façon à obtenir un composé de formule

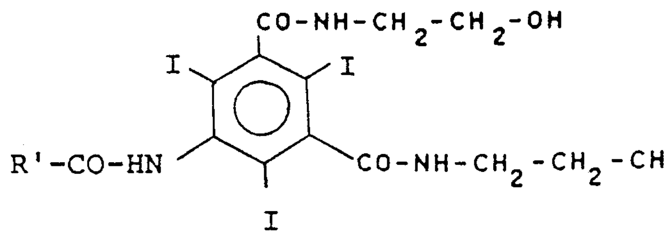

IV

puis soit

c) à faire réagir sur le composé de formule IV du 1-chloro-2,3-propanediol

et

d) à éliminer les groupes protecteurs du groupe R′

soit

c′) à déprotéger le composé de formule IV et

d′) à faire réagir sur le composé déprotégé le 1-chloro-2,3-propanediol.

2. Procédé de préparation d'un produit de contraste, caractérisé en ce qu'il consiste à mettre un composé de formule I tel que défini à la revendication 1 sous une forme administrable.

Office européen des brevets

# RAPPORT DE RECHERCHE EUROPEENNE

Numero de la demande

EP 89 40 1510

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | DE-A-3 429 949 (SCHERING AG)<br>* Revendications 1,15 *<br>--- | 1,4,5 | C 07 C 103/78<br>A 61 K 49/04 |
| X | EP-A-0 015 867 (SCHERING AG)<br>* Revendications 1,8 *<br>--- | 1,4,5 | |
| A | EP-A-0 083 964 (MALLINCKRODT)<br>* Page 1, ligne 1 - page 2, ligne 5; revendications *<br>----- | 1-6 | |
| | | | **DOMAINES TECHNIQUES RECHERCHES (Int. Cl.4)** |
| | | | C 07 C 103/00<br>A 61 K 49/00 |

Le présent rapport a été établi pour toutes les revendications

| Lieu de la recherche | Date d'achèvement de la recherche | Examinateur |
|---|---|---|
| LA HAYE | 05-09-1989 | HELPS I.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)